# EUROPEAN PATENT APPLICATION

(11) **EP 1 881 039 A1**
(43) Date of publication of application: **23.01.2008**
(21) Application number: 05728555.3
(22) Date of filing: 11.04.2005
(51) Int. Cl.: C09C 1/00

(54) **OIL DISPERSION OF POWDER BEING SURFACE-TREATED WITH SPECIFIC ACIDIC ESTER OIL, AND COSMETIC COMPOSITION CONTAINING THE SAME**

(71) Applicant: Miyoshi Kasei, Inc., Saitama-shi, Saitama 336-0975 (JP); The Nisshin OilliO Group, Ltd., Tokyo 104-8285 (JP)
(72) Inventor: HASEGAWA, Yukio, R & D Dept., MIYOSHI KASEI, INC., Saitama-shi, Saitama 336-0975 (JP); SAKURADA, Toru, R & D Dept., MIYOSHI KASEI, INC., Saitama-shi, Saitama 336-0975 (JP); HISHIKAWA, Noboru, THE NISSHIN OILLIO GROUP, LTD., Yokohama-shi, Kanagawa (JP); YAMATO, Yoshihito, THE NISSHIN OILLIO GROUP, LTD., Yokohama-shi, Kanagawa (JP)
(74) Representative: Caen, Thierry Alain
(86) International application number: PCT/JP2005/007016
(87) International publication number: WO 2006/109357

(57) **Abstract**

Provided are a dispersion excellent in the dispersibility of powder particles, dispersion stability, storage stability, and versatility; and a cosmetic blended with such dispersion.
An oil dispersion of a surface-treated powder, comprising a surface-treated powder coated with a half ester oil, and a lipophilic solvent as a dispersion medium, wherein the half ester oil comprises an ester compound having 16 or more carbon atoms in total as can be obtained by reacting at least one kind of alcohols having 1 to 36 carbon atoms with at least one kind of carboxylic acids having 1 to 36 carbon atoms, and wherein the half ester oil has an acid value of 15 or more.

## Description

### TECHNICAL FIELD

The present invention relates to a novel oil dispersion of a powder, which has highly excellent dispersibility of the powder particle, dispersion stability, storage stability, and versatility. More specifically, the present invention relates to an oil dispersion of a powder, comprising at least a surface-treated powder coated with a half ester oil containing a specific half ester compound, and a lipophilic solvent as a dispersion medium of the powder, as well as to a cosmetic composition blended with the oil dispersion. When the oil dispersion of the surface-treated powder is used for a cosmetic production, the process can be in substantial labor savings. In addition, the cosmetics blended with such oil dispersion can get great improvement of feel on use, makeup retention (long wear), and product stability over time. Furthermore, because the oil dispersion has highly excellent dispersibility, the powder is applicable to not only cosmetics but also as powders for use in the fields of, for example, additives for plastics, inks, paints, toners and electronic materials.

### BACKGROUND ART

Recently, in industry of handling powders, there is an increasing need for dispersions as intermediate materials, which are stably dispersed in any dispersion medium (see, for example, Patent Document 1 by the present applicant). The reason is to solve the following problems, for example, a possibility of respiratory disorder of workers caused by inhalation of powders that have been scattered in the work area; a necessity of large storage space for conservation of environment around the work area because the volume of powder increases as the particle size becomes more fine; many technical problems to disperse powders, such as selection of dispersing equipments (machines) and conditions for dispersion, which need much expense in time and effort; and further an improvement of working efficiency by the use of stable dispersion of powder.

On the other hand, one of disadvantages of making powders to an oil dispersion is a poor dispersion stability of powders, which causes a sedimentation or aggregation of powders, so that re-dispersion of powders is necessary when it is used. The oil dispersion of powders is prepared for the purpose of improving various disadvantages of handling powders per se as a raw material (such as the inhalation of dust by workers; capital investment due to setting of dust recovery equipment; time and effort in dispersion; and storage space of powders), and making full use of the functions of the powder. Therefore, favorable composition of powder or favorable selection of dispersion medium is required. It is preferable that an amount of powder in oil dispersion is as high as possible, and also composition of the oil dispersion is simple (one kind of powder and one kind of medium). This is because, when the content of dispersion medium is high, the degree of freedom on a formulation of cosmetics is poor, and versatility for other formulations is impaired.

Furthermore, by the progress in the field of nanotechnology, it is possible to reduce the aggregation of inorganic powders such as ultrafine titanium dioxide and ultrafine zinc oxide, which have a primaryparticle size less than submicron or organic powders by the improvement of technique for surface-treatment, grinding or dispersion. However, since the volume per weight of the powder increases due to a high bulk thereof, a much more storage space is needed. Also, re-aggregation of powder particle is likely to occur, and the scattering ability of powder becomes high, which results in an impaired workability. Under the current techniques, it is extremely difficult to disperse such particles and maintain the stable dispersions. When the microparticles are formulated into cosmetics without dispersing sufficiently, it is difficult to obtain a desired shielding effect against ultraviolet light or infrared radiation, a desired color tone and the like. Recently, inorganic powders are mixed in cosmetics as little as possible to exploit the full potential of the powder, and it is a tendency to reduce powder feel at the time of, or after application. Thus, higher disparsibility of fine grain powder and stability thereof are required. Therefore, various oil dispersions prepared by dispersing fine grain powders into solvents and a method for producing the same are disclosed. For example, a dispersion prepared by dispersing inorganic ultraviolet light filter in liquid ultraviolet light filter (see, for example, Patent Document 2) and a pigment dispersion based on ester oils (see, for example, Patent Document 3) are proposed. In addition, a dispersion containing fine grain powders treated with fatty acids; amino-modified silicone; and silicone type dispersion medium (see, for example, Patent Document 4) and, a method for producing a pigment dispersion for lipstick and a composition of the same (see, for example, Patent Document 5) are proposed. However, these dispersion compositions contain dispersant (s) as an essential component for obtaining a high dispersion and dispersion stability. The present applicant proposed an oil dispersion which has a high versatility to formulation, a high dispersibility and an excellent dispersion stability without any dispersants and any surfactants, in Japanese Patent Kokai Publication JP-A-2002-80748, described above. However, this oil dispersion has some problems in dispersibility of powder and dispersion stability, except when silicone type solvents are used. For these reasons, it is required to further improve the oil dispersion.

[Patent Document 1] JP-P2002-80748A
[Patent Document 2] JP-P2000-26262A
[Patent document 3] JP-P2001-524504A
[Patent document 4] JP-P2001-207060A
[Patent document 5] JP-P2002-363033A

### DISCLOSURE OF THE INVENTION

### PROBLEMS THAT THE INVENTION IS TO SOLVE

It is an object of the present invention to solve the above problems, and to provide a dispersion which has excellent properties of dispersibility of powder particles, dispersion stability, storage stability, and versatility. It is also an object of the present invention to provide a cosmetic having functions of powder superior to conventional products by blending such an excellent dispersion.

### MEANS FOR SOLVING THE PROBLEMS

As a result of perseverant researches towards achieving the above problems, the present inventors have found that, an oil dispersion, comprising at least a surface-treated powder coated with a specific half ester oil, and a lipophilic solvent as a dispersion medium of the powder, has highly excellent dispersibility of powder particles, dispersion stability, storage stability, and versatility, and additionally functions of powder can be fully educed in the cosmetic blended with such an oil dispersion, and such a cosmetic can get great improvement of workability in the production thereof and stability of formulation over time. These findings have led to the completion of the present invention.

That is, the present invention provides an oil dispersion of a surface-treated powder, comprising a surface-treated powder coated with a half ester oil, and a lipophilic solvent, wherein the half ester oil comprises an ester compound having 16 or more carbon atoms in total as can be obtained by reacting at least one kind of alcohols having 1 to 36 carbon atoms with at least one kind of carboxylic acids having 1 to 36 carbon atoms, and wherein the half ester oil has an acid value of 15 or more.

In a preferable embodiment of the present invention, the alcohol of the half ester is monovalent or polyvalent comprising at least one selected from the group consisting of linear carbons, branched carbons, saturated carbons, unsaturated carbons, alicyclic carbons and aromatic rings, while the carboxylic acid of the half ester is monobasic acid or polybasic acid comprising at least one selected from the group consisting of linear carbons, branched carbons, saturated carbons, unsaturated carbons, alicyclic carbons and aromatic rings, or hydroxylic acid thereof.

In a more preferable embodiment of the present invention, the acid value of the half ester oil is 15 or more to 100 or less, while the ratio by weight between a powder to be treated by coating and an agent for treating the surface is 100 : 0.1 to 50. Additionally, it is preferable that content of the surface-treated powder in the oil dispersion is 0.1 to 95 % by weight.

In an alternative aspect of the present invention, there is provided an oil dispersion, comprising at least one kind of a powder, a lipophilic solvent, and the half ester oil as a dispersant or a dispersion stabilizer, and wherein content of the half ester oil is at least 0.1 % by weight.

In a different aspect of the present invention, the oil dispersion of the present invention consists of at least one kind of a powder, and the above half ester oil , and content of the powder is at least 0.1 % by weight therein.

In a still different aspect of the present invention, there is provided a cosmetic containing the oil dispersion at 0.1 to 100 % by weight.

### ADVANTAGE OF THE INVENTION

The oil dispersion of the present invention has highly excellent dispersibility of the powder particle, dispersion stability, storage stability, and versatility because of containing the powder coated with a half ester oil including specific half ester compound(s). Cosmetics blended with such surface-treated powder can get excellent feel on use and makeup retention, and furthermore great improvement of product stability over time.

### PREFERRED EMBODIMENTS FOR CARRYING OUT THE INVENTION

The constitution of the present invention is now described below.
The ester oil for use in coating a powder in accordance with the present invention contains ester compounds having 16 or more carbon atoms in total, as obtained by partially esterifying an alcohol having 1 to 36 carbon atoms with a carboxylic acid having 1 to 36 carbon atoms and has an acid value of 15 or more.

The alcohol having 1 to 36 carbon atoms for use in accordance with the present invention includes monovalent linear alcohols such as methanol, ethanol, propanol, butanol, pentanol, hexanol, heptanol, octanol, nonanol, decanol, undecanol, dodecanol (lauryl alcohol), tridecanol, tetradecanol (myristyl alcohol), pentadecanol, hexadecanol (cetyl alcohol), heptadecanol, octadecanol (stearylalcohol), nonadecanol, eicosanol (arachic alcohol), docosanol (behenyl alcohol), tetracosanol, and setostearyl alcohol; monovalent branched alcohols such as isopropyl alcohol, isobutyl alcohol, isopentyl alcohol, isohexanol, isoheptanol, iso-octanol, dimethyloctanol, isononanol, isodecanol, isoundecanol, isododecanol, isotridecanol, isotetradecanol, isopentadecanol, isohexadecanol (hexyldecanol), isoheptadecanol, isooctadecanol (isostearyl alcohol), isononadecanol, isoeicosanol (octyldodecanol), 2-ethylhexanol, 2-butyloctanol, 2-hexyldecanol, 2-octyldodecanol, 2-decyltetradecanol, 2-dodecylhexadecanol, 2-tetradecyloctadecanol, and 2-hexadecyloctadecanol; monovalent unsaturated alcohols such as undecenol, laurolenol, myristolenol, palmitolenol, oleyl alcohol, elaidyl alcohol, linoleyl alcohol, linolenyl alcohol, erucylalcohol, brassidylalcohol, arachidylalcohol, andjojova alcohol; sterols such as cholesterol, dihydrocholesterol, desmosterol, lanosterol, dihydrolanosterol, agnosterol, latosterol, sitosterol, campesterol, stigmasterol, brassicasterol, ergosterol, phytosterol, and lanolin alcohol; divalent or higher valent alcohols such as ethylene glycol, propylene glycol, trimethylene glycol, butanediol, pentanediol, hexanediol, heptanediol, octanediol, nonanediol, decanediol, pinacol, hydrobenzoin, benzpinacol, cyclopentane-1,2-diol, cyclohexane-1,2-diol, cyclohexane-1,4-diol, dimer diol, hydrogenated dimer diol, neopentylglycol, glycerin, trimethylol propane, trimethylol propane condensate, trimethylol ethane, pentaerythrit, pentaerythrit condensate, sorbit, glycerin condensate, polyethylene glycol, and polypropylene glycol. In accordance with the present invention, these may be used singularly or in mixture of appropriate combinations thereof.

As the carboxylic acid (n=1 to 3) having 1 to 36 carbon atoms for use in accordance with the present invention, any such carboxylic acid of saturated, unsaturated, hydroxyl, aromatic type and the like may be used. For example, monobasic acid (n=1) includes for example linear fatty acids such as formic acid, acetic acid, propionic acid, lactic acid, caproic acid, caprylic acid, capric acid, undecanoic acid, lauric acid, tridecanoic acid, myristic acid, pentadecanoic acid, palmitic acid, margaric acid, stearic acid, nonadecanoic acid, arachidic acid, behenic acid, cerotic acid, montanic acid, and melissic acid; unsaturated fatty acids such as undecylenic acid, palmitoleic acid, oleic acid, linoleic acid, linolenic acid, and arachidonic acid; branched fatty acids such as isooctyl acid (2-ethylhexanoic acid), neotridecanoic acid, isomyristic acid, isopalmitic acid and isostearic acid; and hydroxyfatty acids such as 12-hydroxystearic acid and ricinolic acid. Polybasic acids (n=2 to 3) include for example oxalic acid, malonic acid, succinic acid, cyclobutane-1,1-dicarboxylic acid, cyclohexane-1,2-dicarboxylic acid, phenylene-1,2-diacetic acid, diglycolic acid, dithioglycolic acid, glutaric acid, adipic acid, pimeric acid, suberic acid, azelaic acid, sebacic acid, undecandionoic acid, dodecanedioic acid, eicosandioic acid, octacosandioic acid, 1-,10-decamethylenedioic acid, 1-,12-dodecamethylenedioic acid, 1-,15-pentadecamethylenedioic acid, 1-,28-octacosamethylenedioic acid, 7-ethyloctadecanedioic acid, dimer acid, and hydrogenated dimer acid. In accordance with the present invention, the "dimer acid" means dibasic acid generated by dimerization of an unsaturated fatty acid with 11 to 22 carbon atoms. The unsaturated dibasic acid includes for example fumaric acid, maleic acid and itaconic acid. The hydroxy-polybasic acid includes for example tartaric acid, malic acid, mucic acid, and citric acid. As the hydroxy-polybasic acid, additionally, amino acids and acylated amino acids may also be used. In accordance with the present invention, these may be used singularly or in mixture of an appropriate combination thereof.

The half-ester used in the present invention is a partial ester between the alcohol and the carboxylic acid and can be synthetically prepared by esterification. The esterification can be carried out by charging the individual raw materials in an appropriate reaction container and then reacting them together in the presence or absence of an acid, an alkali or a metal catalyst, preferably in an organic solvent or/and a gas inert to the reaction, at 150 to 200 °C for several hours to about 10 hours, while the byproduct water is eliminated. In case that a catalyst is used, herein, the catalyst is added to 0.001 to 1.0 % of the weight of the reactants. After the completion of the reaction, the product may contain unreactive materials, which are separated and removed by known methods such as rinsing in water, elimination of acids with alkalis, and treatment with adsorbents such as silica gel and are then purified additionally by decoloring and eliminating the odor. The half ester oil thus obtained is in a liquid or solid state at ambient temperature. Preferably, the half ester oil in accordance with the present invention has an acid value of 15 or more and contains an ester compound with 16 or more carbon atoms in total. So as to get the effect of the surface-treated powder of the present invention, more preferably, the acid value of the half ester oil is 15 or more to 100 or less. Inviewof the dispersibilityof the treatedpowdertoa lipophilic solvent, the ester oil then is preferably in liquid, semi-solid or solid with plasticity state at ambient temperature. More preferably, the ester oil is in liquid or in semi-solid state, specifically with a melting point of 70 °C or less.

When the acid value of the ester oil is less than 15, the adsorptivity thereof to a powder base is poor, so that the half ester oil is not used for such surface treatment, causing poor dispersibility to a lipophilic solvent. When the ester compound has less than 16 carbon atoms in total, further, the dispersion stability is poor, and sedimentation or hard-caking is caused in powder particles. Herein, the acid value of the half ester oil in accordance with the present invention can be measured by methods known to persons skilled in the art, for example according to "Kijyun Yushi Bunseki Shiken Houho (Analytical Method of Standard Oils and Fats)" (edited by Japan Oil Chemist's Society).

The amount for surface treatment of the half ester oil with an acid value of 15 or more, which contains such ester compound with total carbon atoms of 16 or more varies depending on the type and specific surface area of an inorganic powder base. The amount is 0.1 to 50 parts by weight to 100 parts by weight of the powder. Preferably, the amount is 0.3 to 35 parts by weight. Such half ester oil for surface treatment may satisfactorily be in mixture of two types or more thereof for the coating treatment. When the amount used for coating is less than 0.1 parts by weight, the surface of the powder particle cannot get a uniformly coated layer and the powder particle cannot become lipophilic so that the dispersibility to a lipophilic solvent is worsened. When the amount is more than 50 parts by weight, the ester oil tends to work to aggregate the powder particle, so that the dispersibility to a lipophilic solvent or dispersion stability cannot be improved. Additionally, it is wasteful.

The half-ester oil is also used favorably as a surface-treating agent for layer B of "MiBrid Dispersion", which is commercially available from Miyoshi Kasei, Inc. and of which the basic technology is described in Japanese Patent Kokai Publication JP-A-2002-80748. A surface-treating agent for layer A is a solid state substance at ambient temperature including a compound(s) selected from among reactive organopolysiloxane, alkylsilane, polyolefin, hydrogenated lecithin (may be in a salt form), N-acyl amino acid (may be in a salt form), fatty acid (may be in a salt form) and dextrin fatty acid ester, and inulin fatty acid ester. A compounding ratio of layer A to layer B is preferably: [treated amount of layer A] < [treated amount of layer B] (% by weight) and the treated amount of layer A is 10% by weight or less.

In the present invention, the half-ester oil can be used as a dispersion medium for powder. Specifically, it is possible to prepare an oil dispersion consisting of either combination of a surface-treated powder coated with a half ester oil, a half ester oil, and a lipophilic solvent, or of an untreated powder, a half ester oil, and a lipophilic solvent.

The powder for use in accordance with the present invention includes not only powders for cosmetics but also powders for use in individual fields of plastic additives, inks, paints, toners, electronic materials and the like. The mean particle size is preferably about 3, 000 µm to 0.001 µm, more preferably about 200 µm to 0.001 µm, most preferably about 100 µm to 0.001 µm. Additionally, the particle size of these powders can be measured as a mean value by a method under observation with optical microscope or electron microscope. The particle size of a particle that is not spherical in shape can be determined as a mean of the total of long diameter, short diameter, thickness and the like.

Inorganic powders, for example, include extender pigments such as mica, sericite, talc, kaolin, synthetic mica, white mica, gold mica, red mica, black mica, lithia mica, calcium carbonate, magnesium carbonate, calcium phosphate, alumina, magnesium oxide,aluminium hydroxide,bariumsulfate,magnesium sulfate, silicic acid, silicic anhydride, magnesium silicate, aluminium silicate, magnesium aluminium silicate, calcium silicate, barium silicate, strontium silicate, silicon carbide, metal salts of tungstic acid, magnesium aluminate, magnesium metasilicate aluminate, chlorohydroxyaluminium, clay, bentonite, zeolite, smectite, hydroxyapatite, ceramic powder, boron nitride, and silica; specific composite extender pigments such as Excel Mica, Excel Pearl and Powder La Vie marketed by Miyoshi Kasei, Inc.; white pigments such as titanium dioxide, zinc oxide and cerium oxide; coloring pigments such as red iron oxide, yellow iron oxide, black iron oxide, chromium oxide, chromium hydroxide, Prussian blue, ultramarine blue, inorganic blue pigments, carbon black, low ordertitanium oxides, mango violet, cobalt violet, tar pigment prepared into the form of lake, and naturally occurring dyes prepared into the form of lake; optically gloss pigments such as bismuth oxychloride, titanium mica, pearl essence, powder prepared by covering synthetic mica with titanium dioxide, powder prepared by covering silica flake with titanium dioxide, which is available under the trade name of "Metashine" from Nippon Sheet Glass Co., Ltd., powder prepared by covering alumina flake with tin oxide and titanium dioxide, powder prepared by covering aluminium flake with titanium dioxide, powder prepared by covering Copper Flake available from Eckert Inc. USA with silica, powder prepared by covering bronze flake with silica, and powder prepared by covering aluminium flake with silica; ultrafine powder of a mean particle size less than 0.1 µm such as ultrafine titanium dioxide, ultrafine zinc oxide, ultrafine iron oxide, and ultrafine cerium oxide; powders with specific particle shapes such as butterfly-shaped barium sulfate, petal-shaped zinc oxide, and nylon fiber of a long diameter of several millimeters; other powders such as luminescent powder marketed under the trade name of "Luminova Series" by Mitsui & Co., Ltd., aluminium powder, stainless powder, tourmaline powder, and amber powder; and the like.

Organic powders includes for example wool powder, polyamide powder, polyester powder, polyethylene powder, polypropylene powder, polystyrene powder, polyurethane powder, benzoguanamine powder, polymethyl benzoguanamine powder, tetrafluoroethylene powder, polymethyl methacrylate powder, cellulose powder, silk powder, silicone powder, silicone rubber powder, synthetic resin powders such as styrene-acrylate copolymer, divinylbenzene-styrene copolymer, vinyl resin, urea resin, phenol resin, fluorine resin, silicone resin, acrylic resin, melamine resin, epoxy resin, and polycarbonate resin, microcrystalline fiber powder, starch powder, acylated lysine powder, long-chain alkylphosphate metal salt powder, metal soap powder, CI pigment Yellow, and CI pigment orange. The tar dye includes for example Red No. 3, Red No.10, Red No.106, Red No.201, Red No.202, Red No.204, Red No.205, Red No.220, Red No.226, Red No.227, Red No.228, Red No.230, Red No.401, Red No.505, Yellow No.4, Yellow No.5, Yellow No.202, Yellow No.203, Yellow No.204, Yellow No.401, Blue No.1, Blue No.2, Blue No . 201, Blue No . 404 , Green No. 3, Green No . 201, Green No . 204, Green No.205, Orange No.201, Orange No.203, Orange No.204, Orange No.206, and Orange No.207. Natural dyes include powders such as carmine, laccaic acid, carsamine, brazilin, and crocin.

Furthermore, the form of the powder to be used may include general powder forms to be blended in cosmetics, such as mixture and composite forms of plural powders and attached form. For example, these powders are prepared in a composite or in a doped form, if necessary, which are then used. Examples thereof include powder prepared by covering inorganic coloring pigments such as red iron oxide with silicic anhydride, powders prepared by covering nylon with white pigments, and powders prepared by covering extender pigments with ultrafine white pigments.

The method for surface-treating powders with the half ester oil in accordance with the present invention includes a step of coating the carboxylic acid moiety as it is in the free form, or a step of treating the carboxylic acid moiety with any of Na, K and polyvalent metals such as Ba, Zn, Ca, Mg, Fe, Zr, Co, Al, and Ti, ammonium or oniums of organic alkanol amines such as monoethanolamine, diethanolamine, triethanolamine, 2-amino-2-methyl-propanol, 2-amino-2-methyl-1,3-propanediol, and triisopropanolamine. Herein, the treatment includes, but is not limited to a surface treatment that is carried out after or simultaneous with substituting the hydrogen atom of the carboxylic acid moiety with another metal or an organic group. Specifically, the treatment includes the following methods. 1. A dry method including a step of mixing a half ester oil with a powder using a Henschel mixer or a super mixer and subsequently drying the resulting mixture. 2. A method including a step of kneading using a kneader or an extruder and subsequent drying. 3. A method including a step of dispersing a powder in water or an organic solvent using a ball mill and a mechano-chemical mill such as a sand grinder and mixing the powder in a half ester oil, and then removing the solvent and drying the resulting powder. 4. A method of making a powder base and a half ester oil in contact to each other in high-speed air stream with for example JET Atomizer for coating the powder. The surface treatment method herein referred to is not limited to these methods described above, as long as any such method is satisfactorily applicable to the surface treatment of powder base.

So as to improve the affinity and adhesion with a surface-treating agent, the powder base for surface treatment may be coated, for example, with at least one of oxides or hydrated oxides of aluminium, calcium, magnesium, cerium, silicone, zirconium, titanium, zinc, iron, cobalt, manganese, nickel and tin. Further, these powder bases may satisfactorily be powders preliminarily treated with known surface-treating agents for example by surface treatment with silicone compounds, surface treatment with acylated amino acids, surface treatment with fatty acids, surface treatment with fluorine compounds, surface treatment with lecithin, surface treatment with polyethylene, surface treatment with alkylsilane, surface treatment with alkyl titanate, surface treatment with ceramide, and surface treatment with dextrin fatty acid ester, inulin fatty acid ester in order to enhance effects for surface treatment more.

The dispersion medium used for an oil dispersion of the present invention may be ether in a liquid or solid state at ambient temperature as long as it is lipophilic. The solvents which can be used in various cosmetics are preferably used. Specifically, the solvents include monovalent alcohol solvents such as methanol, ethanol, propyl alcohol, isopropyl alcohol, butanol, amyl alcohol, hexyl alcohol, heptyl alcohol, octyl alcohol, capryl alcohol, nonyl alcohol, decyl alcohol, phenol, and benzyl alcohol; petroleum hydrocarbons such as normal pentane, normal hexane, normal heptane, normal octane, isohexane, iso-octane, gasoline, and mineral spirit; aromatic hydrocarbon solvents such as benzene, toluene, xylene, cyclohexane, ethylbenzene, and amylbenzene; botanical hydrocarbon solvents such as dipentene, and turpentine oil; nitrohydrocarbon solvents such as nitroparaffin, and nitrobenzene; ketone solvents such as acetone, methyl ethyl ketone, methyl isobutyl ketone, ethyl butyl ketone, and diisobutyl ketone; halogenated hydrocarbon solvents such as methylene chloride, chloroform, carbon tetrachloride, perchloroethylene, and monochlorobenzene; ether solvents such as ethyl ether, isopropyl ether, butyl ether, hexyl ether, propylene oxide, ethyleneglycol monomethyl ether, ethyleneglycol monoethyl ether, and propyleneglycol monomethyl ether; ester solvents such as methyl formate, ethyl formate, butyl formate, methyl acetate, ethyl acetate, butyl acetate, amyl acetate, ethyl propionate, butyl propionate, isobutyl propionate, ethyl butyrate, butyl butyrate, methyl lactate, ethyl lactate, butyl lactate, and amyl lactate; fats and oils such as safflower oil, soybean oil, evening primrose oil, grapeseed oil, rose hip oil, candlenut oil, almond oil, sesame oil, wheat germ oil, corn oil, cottonseed oil, avocado oil, olive oil, camellia oil, persic oil, castor oil, peanut oil, hazelnut oil, macadamia nut oil, meadowfoam oil, cacao butter, shea butter, Japan wax, coconut oil, palm oil, palm kernel oil, sasanqua oil, tea seed oil, beef tallow, horse fat, mink oil, milk fat, egg yolk oil, and turtle oil; hydrocarbon oils suchas liquid (fruid) paraffin, liquidisoparaffin, isododecane, isohexadecane, squalane, squalene, vaseline (petrolatum), paraffin, ceresin, and microcrystalline wax; fatty acids such as lauric acid, myristic acid, palmitic acid, stearic acid, behenic acid, oleic acid, linolic acid, undecylenic acid, hydroxystearic acid, lanolin fatty acid; higher alcohols such as myristyl alcohol, cetyl alchohol, cetostearyl alcohol, stearyl alcohol, aralkyl alcohol, behenyl alcohol, oleyl alcohol, jojoba alcohol, batyl alcohol, cholesterol, phytosterol, lanolin alcohol, and isostearyl alcohol; ester oils such as C12-15 alkyl benzoate, iso-octyl isononanoate, 2-ethylhexyl isononanoate, isononylisononanoate, isotridecyl isononanoate, alkyl (C14, C16, C18) octanoate , cetyl octanoate, isocetyl octanoate, cetostearyl octanoate, stearyl octanoate, isostearyl octanoate, hexyl laurate, isopropyl myristate, myristyl myristate, hexyldecyl myristate, cetyl myristate, octyldodecyl myristate, isostearyl myristate, isopropyl palmitate, 2-ethylhexyl palmitate, octyl palmitate, cetyl palmitate, ethyl stearate, butyl stearate, hexyldecyl stearate, stearyl stearate, ethyl isostearate, isopropyl isostearate, hexyldecyl isostearate, isostearyl isostearate, polyglyceryl isostearate, polyglyceryl diisostearate, polyglyceryl triisostearate, polyglyceryl tetraisostearate, ethyl oleate, oleyl oleate, phytosteryl oleate, octyldodecyl lanolin fatty acid, octyl eicosanoate, fatty acyl (C18-C36) glycol, octyldodecyl lactate, diethoxyethyl succinate, 2-ethylhexyl succinate, dioctyl succinate, diisobutyl adipate, diheptylundecyl adipate, PPG-3 myristyl adipate, PPG-2-myres-10 adipate, propylene glycol dicaprylate, propylene glycol dicaprate, propylene glycol dinonanoate, propylene glycol dicaprylcaprate, propylene glycol distearate, propylene glycol diisostearate, propylene glycol dioleate, neopentyl glycol dioctanoate, neopentyl glycol dicaprate, diisostearyl malate, diethyl sebacate, diisopropyl sebacate, diisopropyl dimer acid, hardened castor oil dimer acid, diisostearyl-phytosteryl dimerdilinoleate, dimerdilinoleyl hydrogenated rosin condensate, dimerdilinoleyl diisostearate, dimerdilinoleyl dimerdilinoleate, trimethylolpropane trioctanoate, trimethylolpropane triisostearate, trioctanoine, glyceryl tricaprylate, glyceryl tricaprylcaprate, glyceryl trimyristate, glyceryl triisostearate, triglyceryl caprylate/caprate/myristate/stearate, glyceryl hydroxystearate, tribehenine, pentaerythrityl tetraoctanoate, pentaerythrit tetraisostearate, pentaerythrityl tetraisostearate, sucrose tetraisostearate, hydroxyalkyl(C12-C14) hydroxydimerdilinoleyl ether, hydroxyalkyl(C16-C18) hydroxydimerdilinoleyl ether, cholesteryl hydroxystearate, dipentaerythrityl hydroxystearate/stearate/rosinate, dipentaerythrityl hydroxystearate/isostearate, dipentaerythrityl hexahydroxystearate, glyceryl octanoate/stearate/adipate, octyldodecyl neopentanoate, isotridecyl neopentanoate, octyldodecyl neopentanoate, myristyl neopentanoate, propylene glycol diisononanoate, glyceryl tri2-ethylhexanoate, trimethylol propane tri2-ethylhexanoate,phytosterylmacadamia nut fatty acid, oligo-ester PPG-7/succinate, diglyceryl oligo-ester hexyldecanoate/sebacate, ethylhexyl calicylate, diphytosteryl-octyldodecylN-lauroyl-L-glutamate, and lauroyl sarcosine isopropyl; waxes such as beeswax, candelilla wax, spermaceti wax, cotton wax, carnauba wax, bayberry wax, bran wax, Chinese wax, orange roughy oil, montan wax, sugar cane wax, shellac wax, lanoline, hard lanoline, reduced lanoline, adsorption-refined lanoline, and jojoba wax; silicones such as methylpolysiloxane, methylphenylpolysiloxane, decamethylcyclotetrasiloxane, alkyl-modified silicone, alcohol-modified silicone, amino-modified silicone, epoxy-modified silicone, olefin-modified silicone, carboxyl-modified silicone, carbinol-modified silicone, phenol-modified silicone, methacryl-modified silicone, mercapto-modifiedsilicone,phosphoric acid-modifiedsilicone, fluorine-modified silicone, higher fatty acid-modified silicone, and polyether-modified silicone; fluoric oil materials (oils) suchasperfluoropolyethel, hydrofluoroether, perfluoromethylcyclopentane, perfluorodimethylcyclohexane, perfluorodimethylcyclobutane, methoxynonafluorobutane, ethoxynonafluorobutane, dodecafluoropentane, tetradecafluorohexane, perfluorodecane, perfluoro-octane, 4-tritrifluoromethylperfluoromorpholine, and 4-pentafluoroethyl perfluoromorpholine; UV absorbing agents. The UV absorbing agents include isostearyl ferulate; benzoic acids such as para-aminobenzoic acid, para-aminobenzoic acid monoglycerin ester, N,N-dimethyl para-aminobenzoic acid ethyl ester, N-N'-diethoxy para-aminobenzoic acid ethyl ester, N-N'-dipropoxy para-aminobenzoic acid ethyl ester; anthranylic acids such as homomenthyl-N-acetyl anthranylate, salicylic acids such as amyl salicylate, menthyl salicylate, homomenthyl salicylate, octyl salicylate, phenyl salicylate, and benzyl salicylate; cinnamic acids such as octyl cinnamate, ethyl-4-isopropyl cinnamate, methyl-2, 5-diisopropyl cinnamate, ethyl-2,4-diisopropyl cinnamate, propyl-p-methoxy cinnamate, isopropyl-p-methoxy cinnamate, isoamyl-p-methoxy cinnamate, octyl-p-methoxy cinnamate,2-ethoxyethyl-p-methoxy cinnamate, and cyclohexyl-p-methoxy cinnamate; benzophenones such as 2,4-dihydroxy benzophenone, 2,2'-dihydroxy-4-methoxy benzophenone, 2-hydroxy-4-methoxy benzophenone, 2-hydroxy-4-methoxy benzophenone-5-sulfonate, 4-phenyl benzophenone, and 4-hydroxy-3-carboxy benzophenone; 3-benzylidene-d, 1-camphor, urocanic acid, urocanic acid ethyl ester, 2-phenyl-5-methylbezoxazole, dibenzalazine, dianisoyl methane, 4-tert-butyl-4'-methoxy dibenzoyl methane, silicone-modified ultraviolet light absorbing agents, and fluorine-modified ultraviolet light absorbing agents. In the present invention, one or more kinds of these can be used as the lipophilic solvent. When the lipophilic solvent used in the present invention is composed of a mixture of plural components, one or more kinds of the components may not be lipophilic as long as a solvent per se obtained by mixing the plural components is lipophilic and uniform.

The oil dispersion of the present invention is preferably composed of a simple composition where possible. The content (amount) of the surface-treated powders coated with the half ester oil is not limited, and the surface-treated powders can be blended in the arbitrary proportion. Incidentally, the preferable amount of the above surface-treated powders is at 1 to 95 % by weight, more preferably at 5 to 90 % by weight. When such an oil dispersion is blended in a cosmetic, the above surface-treated powders and lipophilic solvents, and the other components if necessary, may be directly mixed to produce (prepare) the cosmetic, or after preparing the oil dispersion of the present invention by mixing the above surface-treated powders and lipophilic solvents in advance, the oil dispersion obtained may be used to produce the cosmetic.

The oil dispersion of the present invention may contain other components including third components for the various sakes such as for the sake of enhancing dispersion of coated powder, and stability of the dispersion, and improvement of functions of the oil dispersion due to a synergistic effect of the third components. The components blended for such sake include various surfactants. The surfactants include, for example, anionic surfactants such as alkyl ether sulfate esters such as POE triethanolamine lauryl sulfate; alkyl benzene sulfonates such as sodium dodecyl benzene sulfonate; higher alkyl sulfate esters such as sodium lauryl sulfate, potassium lauryl sulfate; higher fatty acid amido sulfonates such as N-acyl sarcosine, sodium N-myristoyl-N-methyl taurine; higher fatty acid ester sulfate esters such as hardened coconut oil fatty acid glycerine sodium sulfate; higher fatty acid ester sulfonates; higher fatty acid alkylol amido sulfate esters; fatty acid soaps; sulfosuccinates; secondary alcohol sulfate esters; POE alkyl ether carboxylic acid; POE alkyl allyl ether carboxylate; α-olefin sulfonates; sodium lauroyl monoethanol amidosuccinate; diethanol amine N-palmitoyl aspartate; and sodium caseinate; cationic surfactants such as alkyltrimethyl ammonium salts such as stearyl trimethyl ammonium chloride; alkyl pyridinium salts such as distearyl dimethyl ammonium dialkyl dimethyl ammonium chloride; alkyl quaternary ammonium salts; alkyl amine salts; alkyl dimethyl benzyl ammonium salts; alkyl isoquinolinium salts; dialkyl moriphonium (morpholine) salts; POE alkyl amines; polyamine fatty acid derivatives; amyl alcohol fatty acid derivatives; benzalkonium chloride; and benzethonium chloride; imidazoline ampholytic surfactants such as 2-cocoyl-2-imidazolinium hydroxide-1-carboxy ethyloxy 2 sodium salt; betaine ampholytic surfactants such as alkyl betaine, amide betaine, and betaine lauryl dimethyl aminoacetate; nonionic surfactants such as glyceryl fatty acid esters (glyceryl esters of fatty acids) such as glycerine (glyceryl) sesquioleate and glycerine (glyceryl) monostearate; polyglyceryl fatty acid esters such as hexaglyceryl polyricinoleate, diglyceryl monostearate, and decaglyceryl decaoleate; sorbitan fatty acid esters such as sorbitan mono-oleate and sorbitan sesquioleate; propylene glycol fatty acid esters such as propylene glycol monostearate; POE sorbitan fatty acid esters such as POE sorbitan mono-oleate; POE glyceryl fatty acid esters such as POE glycerine triisostearate; POE fatty acid esters such as POE mono-oleate and POE distearate; POE alkyl ethers such as; POE lauryl ether and POE stearyl ether; POE-POPalkyl ethers such as POE-POP hydrogenated lanolin; hardened castor oil; glyceryl alkyl ethers; alkanol amide; sucrose fatty acid esters; dextrin fatty acid esters; inulin fatty acid esters; and hydroxy stearic acid; and the other surfactants suchas ; phospholipids suchas lecithin; glycolipids such as trehalose lipid; fluorine surfactants such as perfluoroalkyl phosphates, perfluoroalkyl sulfonates and perfluoroalkyl carboxylates; copolymers of acrylic acid-alkyl methacrylate; natural or synthetic clay minerals such as bentonite, smectite and kaolin; organic-modified clay minerals such as organic amine cation-modified bentonite; and aerosols.

In the mixing (kneading) or mixing/dispersing method for dispersing the coated powders used in the present invention in dispersion medium, any suitable methods known may be employed.
Examples of such methods in producing oil dispersion are by kneading/mixing machine such as kneader, hemschel kneading machine, roll kneading machine, and extruder kneading machine, and by wet mixing/dispersing equipment such as, propeller mixer, high speed mixer, disperser, homogenizer, ultemizer, fluid-jet-mill, colloid mill, disk grinder, bead mill, sand mill, and basket mil, but not limited to these.

With regard to a method for dispersing the coated powder in the lipophilic solvent (includes one kind of singular solvent or mixture of plural solvents) in the form of a solid state at ambient temperature, it is not particularly difficult. For example, the powder is preferably dispersed in the lipophilic solvent which is in the form of a liquefaction state by heating to temperature the melting point thereof or more. This method is a common method which is conducted for agent form containing much wax in the form of a solid state at ambient temperature or the like, such as lipstick.

Furthermore, in the oil dispersion of the present invention, from the viewpoint of enhancing the effect of the present invention, the only coated powder used in the present invention is preferably used for preparing a dispersion without blending powders and coated powders which have been used up to now. While, the oil dispersion can also be prepared by partially blending the powders, which have been used up to now, within a range without any deterioration of the advantages of the present invention. Or, a process for surface-treating a powder with the half ester oil used in the present invention and a process for preparing an oil dispersion by mixing with a lipophilic solvent can be conducted at the same time.

In the oil dispersion of the present invention, the above half ester oil can be blended with a lipophilic solvent as a dispersant or a dispersion stabilizer for powder, so that a dispersion, which is highly excellent in dispersibility of the powder particle, dispersion stability, storage stability, and suitability for use, can be similarly obtained. In this case, each of the half ester oil, the powder, and the lipophilic solvent may comprise one or more kinds of ingredients. The content of the half ester oil is preferably 0.1 % by weight or more (at least 0.1 % by weight).

In a different embodiment of the present invention, the oil dispersion can be prepared using powder(s)and the half ester oil only. In this case, the content of the powder is preferably 0.1 %by weight or more (at least 0.1 % by weight) . Production of the oil dispersion can be conducted in a similar manner as above.

The cosmetics blended with the oil dispersion of the present invention include, for example, makeup cosmetics such as powder foundation, liquid foundation, cream foundation, oil foundation, stick foundation, pressed powder, face powder, lipstick, lip gloss, rouge, eye shadow, eyebrow pencil, eye liner, mascara, aqueous nail enamel, oil nail enamel, emulsion type nail enamel, enamel top coat, and enamel base coat; fundamental cosmetics such as emollient cream, emollient lotion, milky lotion, massage lotion, cold cream, whitening cream, emulsion, lotion, aesthetic lotion, carmine lotion, cleansing jell, liquid wash, washing foam, washing cream, washing powder, cleansing cosmetics for makeup, and body gloss; hair cosmetics such as hair gloss, hair cream, hair oil, hair shampoo, hair rinse, hair color, and hair brushing cosmetics; and others including cream and emulsions for sun screening and sun tanning, soap, bathing agents, and fragrance.

Cosmetics blended with the oil dispersion of the present invention may appropriately be blended, for example, with pigment dispersants, oils, surfactants, ultraviolet absorbents, preservatives, anti-oxidants, film forming agents, emollient agents, thickeners, dyes, pigments, and fragrance within a range without any deterioration of the advantages of the present invention.

### (Use other than cosmetics)

The oil dispersion of the present invention can be used not only for cosmetics but also for oil dispersions of powder widely used in various fields of additives of plastic products inks, paints, toners (magnetic powders), chemical fibers, materials for packing, electronic materials, and the like. Especially when the powder, which can be used in cosmetics, is used in other fields and the dispersion is likewise required, the above oil dispersion, which are described for cosmetics, can also be employed in the other fields.

### EXAMPLES

The present invention is now described in detail by referring to the following Examples and Comparative Examples, which do not limit the present invention in any way, as a matter of course.

### [Synthetic Example 1 of half ester oil] (Partial ester of monovalent alcohol and dibasic acid)

In a four-necked flask equipped with a stirrer, a thermometer, a tube for purging nitrogen gas, and a water separator are added 2.2 kg of oleyl alcohol, 0.8 kg of succinic acid, and xylene and p-toluenesulfonic acid at 5 % and 0.2 %, respectively to the whole amount charged. Then, the reaction was carried out at 180 °C until a calculated amount of water accumulates in the water separator. After the completion of the reaction, unreactive substances were separated. According to a general method, the resulting reaction product was deodorized, and was then decolored, to obtain a half ester oil at 2.2 kg. The acid value of the resulting ester oil was 43.

### [Synthetic Example 2 of half ester oil] (Partial ester of monovalent alcohol and hydroxy-dibasic acid)

In the same manner as in Synthetic Example 1, 2.0 kg of lauryl alcohol was reacted with 1.0 kg of malic acid. After the completion of the reaction, the resulting product was purified by a general method, to obtain a half ester oil at 2.5 kg. The acid value of the resulting ester oil was 35.

### [Synthetic Example 3 of half ester oil] (Partial ester of divalent alcohol and dibasic acid)

In the same manner as in Synthetic Example 1, 1.2 kg of neopentyl alcohol was reacted with 1.8 kg of adipic acid. After the completion of the reaction, the resulting product was purified by a general method, to obtain a half ester oil at 2.4 kg. The acid value of the resulting ester oil was 75.

### [Synthetic Example 4 of half ester oil] (Ester of tetravalent alcohol and dibasic acid)

In the same manner as in Synthetic Example 1, 1.0 kg of pentaerythrit was reacted with 2.0 kg of azelaic acid. After the completion of the reaction, the resulting product was purified by a general method, to obtain a half ester oil at 2.4 kg. The acid value of the resulting ester oil was 70.

### [Synthetic Example 5 of half ester oil] (Partial ester of monovalent alcohol and polybasic acid)

In the same manner as in Synthetic Example 1, 2.2 kg of isostearyl alcohol was reacted with 0.8 kg of citric acid. After the completion of the reaction, the resulting product was purified by a general method, to obtain a half ester oil at 2.3 kg. The acid value of the resulting ester oil was 88.

### [Synthetic Example 6 of half ester oil] (Partial ester of monovalent alcohol and polybasic acid)

In the same manner as in Synthetic Example 1, 2.0 kg of isostearyl alcohol was reacted with 1.0 kg of citric acid. After the completion of the reaction, the resulting product was purified by a general method, to obtain a half ester oil at 2.2 kg. The acid value of the resulting ester oil was 135.

### [Synthetic Example 7 of half ester oil] (Partial ester of monovalent alcohol and dimer acid)

In the same manner as in Synthetic Example 1, 1.5 kg of isopalmityl alcohol was reacted with 3.4 kg of dimer acid (dibasic acid with 36 carbon atoms, which contains the dimer of oleic acid as the main component). After the completion of the reaction, the resulting product was purified by a general method, to obtain a half ester oil at 3.6 kg. The acid value of the resulting ester oil was 48.

### [Producing Example 1 of oil dispersion]

5 parts by weight of the half ester oil of Synthetic Example 1 and 5 parts by weight of light fluid isoparaffin were added to 100 parts by weight of sericite FSE (manufactured by Sanshin Mining Industry Co., Ltd.), for mixing with a high-speed Henschel mixer for 15 minutes. Subsequently, the resulting mixture was passed through an atomizer, and dried at 105 °C for 8 hours to obtain a half ester oil-treated sericite. 80 parts by weight of the half ester oil-treated sericite and 20 parts by weight of C12-C15 alkyl benzoate (FINSOLV TN: manufactured by FINETEX) were mixed for dispersion (mixed/dispersed) with a homo-mixer for 5 minutes, and subsequently dispersed with a homogenizer for 15 minutes to obtain an oil dispersion.

### [Producing Example 2 of oil dispersion]

100 parts by weight of titanium dioxide CR-50 (manufactured by Ishihara Sangyo Kaisha, Ltd.) and 5 parts by weight of the half ester oil of Synthetic Example 2 are mixed together with a Henschel mixer for 10 minutes. The mixture was ground and treated with a jet mill (manufactured by Hosokawa Micron Corporation), and dried at 105 °C for 8 hours, to obtain a half ester oil-treated titanium dioxide. 2 parts by weight of the half ester oil-treated titanium dioxide, 93 parts by weight of hexyl laurate (Crodamol HL-R: manufactured by Croda Japan), 2.5 parts by weight of coconut oil glucoside and 2.5 parts by weight of polyglyceryl dipolyhydeoxystearate were mixed/dispersed at 90 °C with a homo-mixer for 5 minutes, and subsequently dispersed with a homogenizer to obtain an oil dispersion.

### [Producing Example 3 of oil dispersion]

The same surface treatment as in Example 1 was done, except that the sericite in Example 1 was replaced with yellow iron oxide LL-100P (manufactured by Titan Kogyo Co., Ltd.), and further the half ester oil of Synthetic Example 1 was replaced with 5 parts by weight of the half ester oil of Synthetic Example 3, to obtain a half ester oil-treated yellow iron oxide. 70 parts by weight of the half ester oil-treated yellow iron oxide and 30 parts by weight of diphytosteryl N-lauroyl-L-glutamate (Eldew PS203: manufactured by Ajinomoto Co., Inc.) were mixed/dispersed with a homo-mixer for 5 minutes, and subsequently dispersed by passing through a sand grinder to obtain an oil dispersion.

### [Producing Example 4 of oil dispersion]

The same surface treatment as in Example 1 was done, except that the sericite in Example 1 was replaced with red iron oxide cloisonne (manufactured by Morishita Bengara Co. Ltd.), and further the half ester oil of Synthetic Example 1 was replaced with 6 parts by weight of the half ester oil of Synthetic Example 4, to obtain a half ester oil-treated red iron oxide. 50 parts by weight of the half ester oil-treated red iron oxide and 50 parts by weight of trioctanoine (TIO: manufactured by Nisshin Oillio) were kneaded with a kneader, and subsequently dispersed by subjecting to three rolls processing to obtain an oil dispersion.

### [Producing Example 5 of oil dispersion]

The same surface treatment as in Example 1 was done, except that the sericite in Example 1 was replaced with black iron oxide BL-100P (manufactured by Titan Kogyo Co., Ltd.), and further the half ester oil of Synthetic Example 1 was replaced with 5 parts by weight of the half ester oil of Synthetic Example 5, to obtain a half ester oil-treated black iron oxide. 65 parts by weight of the half ester oil-treated black iron oxide and 35 parts by weight of stearyl-modified acrylsilicone (KP-561P: manufactured by SHIN-ETSU CHEMICAL CO. LTD.) were kneaded with a kneader, and subsequently dispersed by subj ecting to three rolls processing to obtain an oil dispersion.

### [Producing Example 6 of oil dispersion]

100 parts byweight of fattyacid-treatedultrafine titanium dioxide (TTO-S-4: manufactured by Ishihara Sangyo Kaisha, Ltd.) and 10 parts by weight of the half ester oil of Synthetic Example 6 are mixed together with a high-speed Henschel mixer for 20 minutes. The mixture was ground and treated with a jet mill (manufactured by Hosokawa Micron Corporation), and dried at 105 °C for 5 hours, to obtain a half ester oil-treated ultrafine titanium dioxide. 30 parts by weight of the half ester oil-treated ultrafine titanium dioxide, 65 parts by weight of octocrylene (Eusolex OCR: manufactured by MERCK) and 5 parts by weight of aerosol R812 (manufactured by Nippon Aerosol) were mixed/dispersed with a homo-mixer for 5 minutes, and subsequently dispersed with a homogenizer for 15 minutes to obtain an oil dispersion.

### [Producing Example 7 of oil dispersion]

To 100 parts by weight of ultrafine zinc oxide FINEX-50 (manufactured by Sakai Chemical Industry Co., Ltd.) are added 7 parts by weight of the half ester oil of Synthetic Example 7 and 5 parts by weight of silicone oil KF-9901 (manufactured by Shin-etsu Chemical Co., Ltd.), for mixing with a high-speed Henschel mixer for 20 minutes. Subsequently, the resulting mixture was passed through an atomizer, and dried at 105 °C for 6 hours to obtain a half ester oil-treated ultrafine zinc oxide. 60 parts by weight of the half esteroil-treated ultrafine zinc oxide and 40 parts by weight of isohexadecane (Permethyl 101A: manufactured by PRESPERSE) were mixed/dispersed with a homo-mixer for 5 minutes, and subsequently dispersed with a homogenizer for 15 minutes, to obtain an oil dispersion.

### [Producing Example 8 of oil dispersion]

To 100 parts by weight of titanium mica (manufactured by Merck Japan Ltd.; TIMIRON SUPER BLUE) are added 7 parts by weight of the half ester oil of Synthetic Example 6, for mixing with a Henschel mixer for 15 minutes. Subsequently, the resulting mixture was dried at 105 °C for 6 hours to obtain a half ester oil-treated titanium mica. 65 parts by weight of the half ester oil-treated titanium mica and 35 parts by weight of ethyl acetate were mixed/dispersed with a homo-mixer for 20 minutes, to obtain an oil dispersion.

### [Producing Example 9 of oil dispersion]

To 100 parts by weight of Excel Mica JP-2 (manufactured by Miyoshi Kasei Inc.) are added 10 parts by weight of the half ester oil of Synthetic Example 6, for mixing with a Henschel mixer for 10 minutes. Subsequently, the resulting mixture was dried at 105 °C for 5 hours to obtain a half ester oil-treated Excel Mica JP-2. 70 parts byweight of the half ester oil-treated Excel Mica JP-2 and 30 parts by weight of neopentyl glycol dioctanoate were mixed/dispersed with a homo-mixer for 20 minutes, to obtain an oil dispersion.

### [Producing Example 10 of oil dispersion]

With 40 parts by weight of neopentyl glycol di-2-ethylhexanoate is mixed 10 parts by weight of the half ester oil of Synthetic Example 6, for dissolving with a mixer. Next, to the resulting mixture was gradually added 50 parts by weight of ultrafine titanium dioxide (MT-500SA: manufactured by Teika) under agitation with a mixer for 30 minutes, and subsequently dispersed with a sand grinder to obtain an oil dispersion.

### [Producing Example 11 of oil dispersion]

With 50 parts by weight of the half ester oil of Synthetic Example 7 was mixed 50 parts by weight of iron dope ultrafine titanium dioxide (TTO-F-2: manufactured by Ishihara Sangyo Kaisha, Ltd.), with a mixer for 60 minutes, and subsequently dispersed with a sand grinder to obtain an oil dispersion.

### [Comparative Example 1]

According to the method described in Example 6 in Japanese Patent Kokai Publication JP-A-2002-128628, sericite FSE (manufactured by Sanshin Mining Industry Co. , Ltd.) was treated, to obtain a 5 % by weight of dimer dilinoleyl polydimerdilinoleate-5 % by weight treated sericite. 80 parts by weight of the treated sericite and 20 parts by weight of C12-C15 alkyl benzoate (FINSOLV TN: manufactured by FINETEX) were mixed/dispersed with a homo-mixer for 5 minutes, and subsequently dispersed with a homogenizer for 15 minutes to obtain an oil dispersion.

### [Comparative Example 2] (Oil dispersion described in Japanese Patent Kohyo Publication JP-A-2001-524504)

2 parts by weight of titanium dioxide CR-50 (manufactured by Ishihara Sangyo Kaisha, Ltd.) and, 93 parts by weight of hexyl laurate (Crodamol HL-R: manufactured by Croda Japan), 2.5 parts by weight of coconut oil glucoside and 2.5 parts by weight of polyglyceryl dipolyhydeoxystearate were mixed/dispersed at 90 °C with a homo-mixer for 5 minutes, and subsequently dispersed with a homogenizer to obtain an oil dispersion.

### [Comparative Example 3]

According to the method described in Example 4 in Japanese Patent Kokai Publication JP-A-2002-188024, yellow iron oxide LL-100P (manufactured by Titan Kogyo Co., Ltd.) was treated, to obtain a inulin stearate ester-treated yellow iron oxide. 70 parts by weight of the treated yellow iron oxide and 30 parts by weight of diphytosteryl N-lauroyl-L-glutamate (Eldew PS203: manufactured by Ajinomoto Co., Inc.) weremixed/dispersed with a homo-mixer for 5 minutes, and subsequently dispersed by passing through a sand grinder to obtain an oil dispersion.

### [Comparative Example 4] (Oil dispersion described in Japanese Patent Kokai Publication JP-A-2002-363033)

50 parts byweight of red iron oxide cloisonne (manufactured by Morishita Bengara Co. Ltd.), 20 parts by weight of hexadecene copolymer and 50 parts by weight of trioctanoine (TIO: manufactured by Nisshin Oillio) were kneaded with a kneader, and subsequently dispersed by subjecting to three rolls processing to obtain an oil dispersion.

### [Comparative Example 5]

According to the method described in Example 1 in Japanese Patent Kokai Publication JP-A-H05-339518, black iron oxide BL-100P (manufactured by Titan Kogyo Co., Ltd.) was treated, to obtain a dimethyl polysiloxane-5 % by weight treated black iron oxide. 65 parts by weight of the treated black iron oxide and 35 parts by weight of stearyl-modified acrylsilicone (KP-561P: manufactured by SHIN-ETSU CHEMICAL CO. LTD.) were kneaded with a kneader, and subsequently dispersed by subj ecting to three rolls processing to obtain an oil dispersion.

### [Comparative Example 6] (Production of oil dispersion described in Japanese Patent Kokai Publication JP-A-2000-26262)

30 parts by weight of fatty acid-treated ultrafine titanium dioxide (TTO-S-4: manufactured by Ishihara Sangyo Kaisha, Ltd.), and 65 parts by weight of octocrylene (Eusolex OCR: manufactured by MERCK) and 5 parts by weight of aerosol R812 (Germany: manufactured by Degussa) were mixed/dispersed with a homo-mixer for 5 minutes, and subsequently dispersed with a homogenizer for 15 minutes to obtain an oil dispersion.

### [Comparative Example 7]

According to the method described in Example 16 in Japanese Patent Kohyo Publication JP-A-2002-516602, ultrafine zinc oxide FINEX-50 (manufactured by Sakai Chemical Industry Co., Ltd.) was treated to obtain a silicone polymer-10 % by weight treated ultrafine zinc oxide. 60 parts by weight of the treated ultrafine zinc oxide and 40 parts by weight of isohexadecane (Permethyl 101A: manufactured by PRESPERSE) were mixed/dispersed with a homo-mixer for 5 minutes, and subsequently dispersed with a homogenizer for 15 minutes, to obtain an oil dispersion.

### [Comparative Example 8]

According to the method described in producing Example 1 in Japanese Patent Kokai Publication JP-A-2001-181136, titanium mica (manufactured by Merck Japan Ltd. ; TIMIRON SUPER BLUE) was treated to obtain a N-octyl triethoxysilane-7 % by weight treated titanium mica. 65 parts by weight of the treated titanium mica and 35 parts by weight of ethyl acetate were mixed/dispersed with a homo-mixer for 20 minutes, to obtain an oil dispersion.

### [Comparative Example 9]

According to the method described in Example 3-1 in Japanese Patent Kokai Publication JP-A-2000-212041, 100 parts by weight of Excel Mica JP-2 (manufactured by Miyoshi Kasei Inc.) was treated to obtain a zinc N-palmitoyl silk amino acid-5 % by weight coated Excel Mica JP-2. 70 parts by weight of the treated Excel Mica JP-2 and 30 parts by weight of neopentyl glycol dioctanoate were mixed/dispersed with a homo-mixer for 20 minutes, to obtain an oil dispersion.

The oil dispersion comprising the half ester oil-treated powders in accordance with the present invention and a lipophilic solvent, and the treated powders in Comparative Examples, as obtained as described above, were evaluated by the following test methods. The results are shown in Table 1.

### (1) Particle size of powder in oil dispersion

Themeanparticlesize (D50: µm) of powder in each dispersion was determined using a laser diffraction particle size apparatus (manufactured by Shimazu Seisakusyo: SALD-2000J) which is a high concentration particle size apparatus.

### (2) Dispersion stability with lapse of time

Each dispersion was put in a 50 cc of transparent glass vial with a collection cap and was allowed to stand in a oven at 50 °C over an one-month period to observe the state of solid-liquid separation thereof (stable: ○, separated: ×).

### (3) Particle size of powder in oil dispersion with lapse of time

Mean particle size (D50) of powder in each of the samples in (2) described above was determined by the method in (1) described above.

**Table 1**

| Samples | D50 (µm) | Stability with lapse of time | D50 with lapse of time |
|---|---|---|---|
| Producing Example 1 | 5.12 | ○ | 5.17 |
| Producing Example 2 | 0.38 | ○ | 0.39 |
| Producing Example 3 | 0.46 | ○ | 0.49 |
| Producing Example 4 | 0.42 | ○ | 0.42 |
| Producing Example 5 | 0.45 | ○ | 0.47 |
| Producing Example 6 | 0.11 | ○ | 0.19 |
| Producing Example 7 | 0.13 | ○ | 0.18 |
| Producing Example 8 | 12.56 | ○ | 12.67 |
| Producing Example 9 | 9.62 | ○ | 9.75 |
| Producing Example 10 | 0.13 | ○ | 0.13 |
| Producing Example 11 | 0.25 | ○ | 0.25 |
| Comparative Example 1 | 6.23 | × | 7.55 |
| Comparative Example 2 | 0.57 | × | 0.99 |
| Comparative Example 3 | 0.66 | × | 1.07 |
| Comparative Example 4 | 0.78 | × | 1.56 |
| Comparative Example 5 | 0.97 | × | 1.73 |
| Comparative Example 6 | 0.50 | × | 1.16 |
| Comparative Example 7 | 0.89 | × | 1.86 |
| Comparative Example 8 | 16.32 | × | 17.58 |
| Comparative Example 9 | 12.75 | × | 14.28 |

As shown in Table 1, the oil dispersions of the present invention have highly excellent dispersibility and dispersion stability in comparison to that of the prior art. In addition, from Table 1, it was found that the oil dispersions of the present invention have particularly excellent dispersibility and dispersion stability when the acid value of the half ester oil therein is 15 or more to 100 or less.

Then, the oil dispersions of the present invention were blended in individual cosmetics to verify the effects thereof, compared with oil dispersions of the prior art.

### [Example 1] (2-way powder foundation)

A 2-way powder foundation of the composition shown in Table 2 was produced by the following method.

**Table 2**

| | |
|---|---|
| 1. Dimethicone-treated red iron oxide: | 0.9 |
| 2. Dimethicone-treated Yellow iron oxide: | 2.5 |
| 3. Dimethicone-treated Black iron oxide: | 0.3 |
| 4. Dimethicone-treated Titanium dioxide: | 12.0 |
| 5. Mica: | 6.0 |
| 6. Talc: | qs. |
| 7. Sericite oil dispersion (Producing Example 1 or Comparative Example 1): | 25.0 |
| 8. Glyceryl trioctanoate: | 4.0 |
| 9. Fluid paraffin: | 1.2 |
| 10. Methylphenylpolysiloxane: | 1.0 |
| 11. Preservative: | appropriate amount |
| 12. Fragrance: | appropriate amount |

### (Production Process)

(1) The components 1 through 6 were mixed together and uniformly ground.
(2) The components 7 through 12 were uniformly dissolved.
(3) The mixture obtained above in (2) was added to the mixture obtained above in (1), for grinding and sieving. Then, the mixture thus obtained was pressed to obtain a 2-way powder foundation.

The powder foundation of the Example as obtained above has good feel on use, and excellent makeup retention, excellent stabilities with temperature changes and lapse of time in comparison to that of the Comparative Example.

### [Example 2] (Eye shadow)

An eye shadow of the composition shown in Table 3 was prepared by the following method.

**Table 3**

| | |
|---|---|
| 1. Dimethicone-treated chromic oxide | 6.5 |
| 2. Dimethicone-treated ultramarine blue | 4.8 |
| 3. Titanium mica | 9.0 |
| 4. Excel Mica oil dispersion (Producing Example 9 or Comparative Example 9) | 20.0 |
| 5. Talc | qs. |
| 6. Silica bead | 6.0 |
| 7. Vasseline | 5.5 |
| 8. Malic acid diisostearyl (diisostearyl malate) | 4.0 |
| 9. Squalane | 1.5 |
| 10. Methylphenylpolysiloxane | 1.5 |
| 11. Preservative | appropriate amount |
| 12. Fragrance | appropriate amount |

### (Production Process)

(1) The components 1 through 6 were mixed together and uniformly ground.
(2) The components 7 through 12 were uniformly dissolved.
(3) The mixture obtained above in (2) was added to the mixture obtained above in (1), for grinding and sieving. Then, the mixture thus obtained was pressed to obtain an eye shadow.

The eye shadow of the Example as obtained above has good adhesion, and excellent resistance to a change in temperature and stability with lapse of time in comparison to that of the Comparative Example.

### [Example 3] (Emulsion-type foundation)

An emulsion type foundation of the composition shown in Table 4 was prepared by the following method.

**Table 4**

| | |
|---|---|
| 1. Decamethylcyclopentasiloxane | 50.0 |
| 2. Dimer acid hardened castor oil | 4.0 |
| 3. Octadecyl dimethyl benzyl ammonium salt-modified montmorillonite | 4.5 |
| 4. Dimethylpolysiloxane polyoxyalkylene polymer (HLB= 4.5) | 2.5 |
| 5. SA-SB-300 (manufactured by Miyoshi Kasei, Inc.) | 6.0 |
| 6. Sericite oil dispersion (Producing Example 1 or Comparative Example 1) | 1.6 |
| 7. Titanium dioxide oil dispersion (Producing Example 2 or Comparative Example 2) | 30.0 |
| 8. Yellow iron oxide oil dispersion (Producing Example 3 or Comparative Example 3) | 4.3 |
| 9. Red iron oxide oil dispersion (Producing Example 4 or Comparative Example 4) | 3.5 |
| 10. Black iron oxide oil dispersion (Producing Example 5 or Comparative Example 5) | 1.0 |
| 11. Ethanol | 7.0 |
| 12. Propylene glycol | 3.0 |
| 13. Sodium chloride | 2.0 |
| 14. Purified water | qs. |
| 15. Preservative | appropriate amount |
| 16. Fragrance | appropriate amount |

### (Production Process)

(1) The components 1 through 6 were mixed together and dissolved together, to which the components 7 through 10 were added for uniform dispersion.
(2) The components 11 through 15 were dissolved under heating.
(3) The component mixture obtained above in (2) was gradually added to the component mixture obtained above in (1), for emulsification and subsequent cooling. Then, component 16 was added to the resulting cooled mixture to obtain an emulsion type foundation.

The emulsion type foundation of the Example as obtained above has good coloring ability and feel on use, excellent makeup retention, excellent stabilities with temperature changes and lapse of time in comparison to that of the Comparative Example.

### [Example 4] (Eyeliner)

An eyeliner of the composition shown in Table 5 was prepared by the following method.

**Table 5**

| | |
|---|---|
| 1. Black iron oxide oil dispersion (Producing Example 5 or Comparative Example 5) | 23.0 |
| 2. Titanium dioxide oil dispersion (Producing Example 2 or Comparative Example 2) | 30.0 |
| 3. Decamethylcyclopentasiloxane | 25.0 |
| 4. Jojoba oil | 3.5 |
| 5. Polyether-modified silicone (HLB = 4.5) | 1.5 |
| 6. Ethanol | 2.5 |
| 7. Purified water | qs. |
| 8. Preservative | 3.5 |
| 9. Fragrance | appropriate amount |

### (Production Process)

(1) The components 1 through 5 were mixed together under warming for uniform dispersion.
(2) The components 6 through 8 were dissolved under warming.
(3) The component mixture obtained above in (2) was gradually added to the component mixture obtained above in (1), for emulsification and subsequent cooling. Then, component 9 was added to the resulting cooled mixture to obtain an eyeliner.

The eyeliner of the Example as obtained above has good adhesion, and excellent stabilities with temperature changes and lapse of time in comparison to that of the Comparative Example.

### [Example 5] (Emollient cream)

An emollient cream of the composition shown in Table 6 was prepared by the following method.

**Table 6**

| | |
|---|---|
| 1. Dimethylpolysiloxane 6cs | 7.0 |
| 2. Methylphenylpolysiloxane | 5.0 |
| 3. Squalane | 5.0 |
| 4. Neopentyl glycol dioctanoate | 4.5 |
| 5. Polyether-modified silicone (HLB = 4.5) | 3.5 |
| 6. Nylon bead | 5.0 |
| 7. Yellow iron oxide oil dispersion (Producing Example 3 or Comparative Example 3) | 0.4 |
| 8. Magnesium sulfate | 0.7 |
| 9. Glycerin | 10.0 |
| 10. Preservative | appropriate amount. |
| 11. Purified water | qs. |
| 12. Fragrance | appropriate amount |

### (Production Process)

(1) The components 1 through 5 were mixed together and dissolved together, to which the components 6 and 7 were added for uniform dispersion.
(2) The components 8 through 11 were dissolved under heating.
(3) The component mixture obtained above in (2) was gradually added to the component mixture obtained above in (1), for emulsification and subsequent cooling. Then, component 12 was added to the resulting cooled mixture to obtain an emollient cream.

The emollient cream of the Example as obtained above has good coloring ability and feel on use, and excellent stabilities with temperature changes and lapse of time in comparison to that of the Comparative Example.

### [Example 6] (Two-layer type sun screening lotion)

A sun screening lotion of the composition shown in Table 7 was prepared by the following method.

**Table 7**

| | |
|---|---|
| 1. Decamethylcyclopentasiloxane | 15.0 |
| 2. Isotridecyl Isononanoate | 5.0 |
| 3. Macadamia nut oil | 4.0 |
| 4. Glyceryl monoisostearate | 3.5 |
| 5. Polyether-modified silicone (HLB = 3.5) | 2.0 |
| 6. Ultrafine titanium dioxide oil dispersion (Producing Example 6 or Comparative Example 6) | 15.0 |
| 7. Ultrafine zinc oxide oil dispersion (Producing Examples 7 or Comparative Example 7) | 15.0 |
| 8. Glycerin | 8.0 |
| 9. Sodium citrate | 0.3 |
| 10. Tetrasodium edetate | 0.1 |
| 11. Preservative | appropriate |
| | amount |
| 12. Purified water | qs. |
| 13. Fragrance | appropriate |
| | amount |

### (Production Process)

(1) The components 1 through 5 were mixed together and dissolved together, to which the components 6 and 7 were added for uniform dispersion.
(2) The components 8 through 12 were dissolved under heating.
(3) The component mixture obtained above in (2) was gradually added to the component mixture obtained above in (1), for emulsification and subsequent cooling. Then, component 13 was added to the resulting cooledmixture to obtain a sun screening lotion.

The sun screening lotion of the Example as obtained above has both high protective effect against ultraviolet light (ultraviolet light protective effect) and good feel on use, and excellent stabilities with temperature changes and lapse of time in comparison to that of the Comparative Example.

### [Example 7] (Lipstick)

A lipstick of the composition shown in Table 8 was prepared by the following method.

**Table 8**

| | |
|---|---|
| 1. Diisostearyl malate | 15.0 |
| 2. Glyceryl triisooctanoate | qs. |
| 3. Glutamic acid phytosterol octyldodecanol | 15.0 |
| 4. Carnauba wax | 4.5 |
| 5. Candelilla wax | 5.0 |
| 6. Ceresin wax | 5.0 |
| 7. Titanium dioxide oil dispersion (Producing Example 2 or Comparative Example 2) | 30.0 |
| 8. Red iron oxide oily dispersion (Producing Example 4 or Comparative Example 4) | 13.8 |
| 9. Anti-oxidant | 0.1 |
| 10. Preservative | appropriate amount |

### (Production Process)

(1) The components 1 through 6 and the components 9 and 10 were heated for uniform dissolution.
(2) The components 7 and 8 were added to the component mixture obtained above in (1) for uniform dispersion.
(3) After deaeration, the resulting mixture was poured in a mold, for cooling. Then, the resulting cooled mixture was subjected to flaming to obtain a lipstick.

The lipstick of the Example as obtained above has good coloring ability, luster (gloss) and spreadability, and excellent stabilities with temperature changes and lapse of time in comparison to that of the Comparative Example.

### [Example 8] (Preparation of Nail enamel)

A nail enamel of the composition shown in Table 9 was prepared by the following method.

**Table 9**

| | |
|---|---|
| 1. Nitrocellulose | 15.0 |
| 2. Sucrose benzoate | 5.0 |
| 3. Alkyd resin | 7.0 |
| 4. Toluenesulfonic acid amide resin | 4.0 |
| 5. Organic-modified bentonite | 2.5 |
| 6. Camphor | 2.5 |
| 7. Ethyl acetate | qs. |
| 8. Butyl acetate | 15.0 |
| 9. Isopropyl alcohol | 5.0 |
| 10. Titanium mica oil dispersion (Producing Example 8 or Comparative Example 8) | 12.0 |

### (Production Process)

(1) The components 1 through 9 were mixed, dispersed and dissolved together, uniformly.
(2) The component 10 was added to the component mixture obtained above in (1) to obtain a nail enamel.

The nail enamel of the Example as obtained above has excellent bright (optically gloss) feel, and stabilities with temperature changes and lapse of time in comparison to that of the Comparative Example.

### [Example 9] (Eyeliner)

An eyeliner of the composition shown in Table 10 was prepared by the following method.

**Table 10**

| | |
|---|---|
| 1. Excel Mica oil dispersion (Producing Example 9 or Comparative Example 9) | 3.5 |
| 2. Black iron oxide oil dispersion (Producing Example 5 or Comparative Example 5) | 6.5 |
| 3. Organic-modified bentonite | 0.5 |
| 4. Light fluid isoparaffin | 67.1 |
| 5. Carnauba wax | 4.5 |
| 6. Beeswax | 1.0 |
| 7. Microcrystalline wax | 11.0 |
| 8. Vaseline | 2.0 |
| 9. Anti-oxidant | 0.2 |
| 10. Preservative | 0.2 |

### (Production Process)

(1) The components 3 through 10 were mixed together for uniform dispersion under heating.
(2) The components 1 and 2 were added to the component mixture obtained above in (1) . The resulting mixture was cooled under agitation, to obtain an eyeliner.

The eyeliner of the Example as obtained above has good adhesion, and excellent stabilities with temperature changes and lapse of time in comparison to that of the Comparative Example.

### [Example 10] (Washing foam)

A washing foam of the composition shown in Table 11 was prepared by the following method.

**Table 11**

| | |
|---|---|
| 1. Excel Mica oil dispersion (Producing Example 9 or Comparative Example 9) | 2.5 |
| 2. Stearic acid | 1.5 |
| 3. Lauroyl monosodium glutamate | 17.5 |
| 4. 1,3 butylene glycol | 25.0 |
| 5. PEG 20000 | 13.0 |
| 6. Purified water | qs. |

### (Production Process)

(1) The components 2 through 4 were mixed for dissolution.
(2) The component 1 was dispersed in the component mixture obtained above in (1).
(3) The component 5 was added to the component 6 under heating. Then, the resulting mixture was added to the component mixture obtained above in (2), to obtain a washing foam.

The washing foam of the Example as obtained above has good feel on use without tightness after washing, and excellent stabilities with temperature changes and lapse of time in comparison to that of the Comparative Example.

### [Example 11] (Body shampoo)

A body shampoo of the composition shown in Table 12 was prepared by the following method.

**Table 12**

| | |
|---|---|
| 1. Sericite oil dispersion (Producing Example 1 or Comparative Example 1) | 5.0 |
| 2. Coconut oil fatty acid glutamic acid triethanolamine | 25.0 |
| 3. Coco amide propyl betaine | 4.0 |
| 4. Panthenol | 1.0 |
| 5. Concentrated glycerin | 4.0 |
| 6. PCA soda | 1.0 |
| 7. Purified water | qs. |

### (Production Process)

(1) The components 2-6 were mixed for dissolution.
(2) The components 1 and 7 were added to the mixture above (1).
   The resulting mixture was mixed well to obtain a body shampoo.

The body shampoo of the Example as obtained above has good feel on use without tightness after washing, and excellent stabilities with temperature changes and lapse of time in comparison to that of the Comparative Example.

### [Example 12] (oil sun screening cosmetic)

An oil sun screening cosmetic of the composition shown in Table 13 was prepared by the following method.

**Table 13**

| | |
|---|---|
| 1. p-methoxy cinnamate-2-ethylhexyl | 6.0 |
| 2. Polyglyceryl tri-isostearate | 15.0 |
| 3. Isotridecyl myristate | 27.0 |
| 4. Oil dispersion of Producing Example 10 | 20.0 |
| 5. Oil dispersion of Producing Example 11 | 3.5 |
| 6. Polyether-modified silicone (HLB = 4.5) | 1.5 |
| 7. Purified water | qs. |

### (Production Process)

(1) The components 1 through 6 was mixed for dissolution.
(2) The component 7 was added to the component mixture above in (1). The resulting mixture was mixed well to obtain an oil sun screening cosmetic.

The oil sun screening cosmetic of the Example as obtained above has excellent feel on use, and excellent stabilities with temperature changes and lapse of time.

## Claims

1. An oil dispersion of a powder, said dispersion comprising a surface-treated powder coated with a half ester oil, and a lipophilic solvent as a dispersion medium, wherein the half ester oil comprises an ester compound having 16 or more carbon atoms in total as can be obtained by reacting one or more alcohols having 1 to 36 carbon atoms with one or more carboxylic acids having 1 to 36 carbon atoms, and wherein the half ester oil has an acid value of 15 or more.

2. The oil dispersion of claim 1, wherein the alcohol is monovalent or polyvalent comprising at least one selected from the group consisting of linear carbons, branched carbons, saturated carbons, unsaturated carbons, alicyclic carbons and aromatic rings.

3. The oil dispersion of claim 1 or 2, wherein the carboxylic acid is monobasic acid or polybasic acid comprising at least one selected from the group consisting of linear carbons, branched carbons, saturated carbons, unsaturated carbons, alicyclic carbons and aromatic rings, or hydroxylic acid thereof.

4. The oil dispersion of any one of claims 1 to 3, wherein the acid value of the half ester oil is 15 or more to 100 or less.

5. The oil dispersion of any one of claims 1 to 4, wherein content of the surface-treated powder is 0.1 to 95 % by weight.

6. An oil dispersion of a powder comprising
one or more kinds of powders;
a half ester oil that comprises an ester compound having 16 or more carbon atoms in total as can be obtained by reacting one or more alcohols having 1 to 36 carbon atoms with one or more carboxylic acids having 1 to 36 carbon atoms, wherein the half ester oil has an acid value of 15 or more; and
a lipophilic solvent.

7. The oil dispersion of claim 6, wherein content of the half ester oil is at least 0.1 % by weight.

8. An oil dispersion consisting of
one or more kinds of powders;
a half ester oil that comprises an ester compound having 16 or more carbon atoms in total as can be obtained by reacting one or more alcohols having 1 to 36 carbon atoms with one or more carboxylic acids having 1 to 36 carbon atoms, wherein the half ester oil has an acid value of 15 or more; and
a lipophilic solvent;
wherein content of the powder in the dispersion is at least 0.1 % by weight.

9. A cosmetic comprising the oil dispersion of any one of claims 1 to 8 in an amount from 0.1 to 100 % by weight.
